# EUROPEAN PATENT SPECIFICATION

(11) **EP 2 474 335 B1**
(45) Date of publication and mention of the grant of the patent: **14.10.2015**
(21) Application number: 11150201.9
(22) Date of filing: 05.01.2011
(51) Int. Cl.: A61M 16/06

(54) **Improved cushion for facial mask for treating sleep disorders**
Verbessertes Kissen für Gesichtsmaske zur Behandlung von Schlafstörungen
Coussin amélioré pour masque facial afin de traiter les troubles du sommeil

(43) Date of publication of application: 11.07.2012
(73) Proprietor: Air Liquide Medical Systems, 92160 Antony (FR)
(72) Inventor: Alberici, Luca, 25128, Brescia (IT); Rivetti, Alberto, Rodengo Saiano (BS) 25050, Rovato (BS) (IT); Sandoni, Giuseppe, 25124, Brescia (IT)
(74) Representative: Pittis, Olivier

(56) References cited:
- EP-A- 1 841 481
- EP-A1- 1 258 266
- EP-A2- 1 982 740
- WO-A1-2009/100905
- US-A1- 2003 196 658

## Description

The invention concerns a cushion for a respiratory mask, in particular a facial mask, and a respiratory mask equipped with such a cushion for use in the treatment of respiratory conditions or diseases, such as obstructive sleep apnea, said cushion being made of soft material and comprising two superimposed flexible membranes ensuring efficient air tightness.

Masks, such as facial or nasal masks, are commonly used for delivering non-invasive positive pressure ventilation (NPPV) or for nasal continuous positive airway pressure (N-CPAP) therapy in sleep disordered breathing (SDB) conditions, such as obstructive sleep apnea (OSA). They typically deliver a flow of breathable gas for, or to assist in, patient respiration, especially during the night, i.e., when the patient is sleeping. Such a mask assembly typically comprises a rigid or semi-rigid hollow shell, also called a mask body, defining a breathing chamber that receives at least a part of the patient's nose and/or mouth, and further comprising a soft face-contacting cushion that comes into contact with the patient's face, and an optionally pivotable forehead support and a headgear for correctly positioning, maintaining and/or securing the mask on the head of a patient.

The shell of the mask or mask body is connected to a gas supply line which delivers a respiratory gas, such as air under pressure, into the breathing chamber of the shell through a gas opening or inlet orifice arranged in the wall of the mask body. The connection between the gas line and the mask body is usually obtained by means of a hollow connector comprising an internal passage for the gas. The connector can have an elbow-shape, i.e. be curved, or any other shape, and can also be rotatable around the axis of the inlet orifice. The soft face-contacting cushion often comprises two superimposed membranes, e.g. an inner membrane recovered by an outer membrane that ensures the air tightness all around the periphery of the cushion in contact with the user's face. These types of respiratory masks are known from documents such asEP-A-1737524, EP-A-1841481, EP-A-956069 or EP-A-1118346.

For instance, document EP-A-1841481 or WO-A-2006/074513 discloses a cushion for a facial respiratory mask that comprises an inner membrane and an outer membrane. The thickness of these membranes varies between a nasal bridge region and a chin region of the cushion.

Further, EP-A-1982740 teaches a facial mask with a cushion that is similar to the one of EP-A-1841481 as it does not comprise any inner membrane in the nasal bridge region. In said region, the inner membrane has been cut out.

Furthermore, EP-A-1258266 discloses a cushion for nasal mask comprising three or more superimposed membranes. The lower part of the cushion does not contact the chin region of the user, but the lip region that has a different morphology. Further, this document advocates that the inner membrane in the nasal bridge region should be thinner, for instance of 0.508 mm, than in the lip region, for instance of 1,524 mm, and that the outer membrane is a same thickness in the nasal bridge region and in the lip region.

More precisely, when the mask is carried by the patient, the outer membrane comes into contact with the different regions of the user's face, i.e. the nasal bridge region, the chin region and the two lateral cheek regions that join the nasal bridge region to the chin region. As both membranes are made of a resilient flexible or soft material, the membranes are deformed under the traction forces exerted by the headgear of the mask, so as to match the profile and forms of the user's face, thereby ensuring the air tightness of the mask.

However, the existing masks equipped with such two-membrane cushions are not totally satisfying. A problem that often exists is that leaks appear in the nasal bridge region, during the night while the patient is sleeping, thus allowing for undesirable and unpleasant escape of the gas under pressure.

Those loses of gas under pressure negatively impact the efficiency of the overall treatment and further cause some comfort issues for the patient, especially since due to the leaks some of the gas under pressure, such as air, is blown directly toward the patient's eyes thereby creating significant discomfort.

Even though the problem is well known, controlling these air leaks is not an easy task as they depend on the patient's face shape, i.e. the patient's physiognomy, especially the width of the patient's nose. In other words, the leaks will vary from one patient to another.

Hence, the problem to be solved is to provide an improved cushion for respiratory masks, especially for facial masks, equipped in a manner to limit the gas leaks in the nasal region when a patient is using the mask while sleeping, regardless of the patient's nasal bridge region profile or physiognomy. In other words, the goal is to propose an improved cushion for respiratory masks that is able to ensure an efficient gas tightness whatever the patient's physiognomy, i.e. a cushion that can match various face shapes.

The solution of the present invention concerns a cushion for respiratory mask, such as facial masks, having a three-dimensional shape with a central passage for receiving at least a part of the nose and/or mouth of a user, said cushion comprising at least a nasal bridge region, two cheek regions and a chin region, which come into contact respectively with the nasal bridge region, the cheek regions and the chin region of the user when the cushion is positioned on the user's face, said cushion comprising an inner membrane and an outer membrane, said inner and outer membranes being superimposed so that the outer membrane recovers the inner membrane and is in direct contact with the face of the user, said inner and outer membranes being made of a flexible material, characterized in that the outer membrane has, in the nasal bridge region of the cushion, a first thickness that is less than the second thickness of said outer membrane in the chin region and/or the inner membrane has, in the nasal bridge region of the cushion, a third thickness that is more than the fourth thickness of said outer membrane in the chin region.

The third thickness is at least 0,8 mm and the fourth thickness is less than 0,8 mm.

The cushion for respiratory masks according to the present invention can further comprise one or more of the following additional features:
- the inner and outer membranes are arranged on a cushion frame.
- the first and second thickness are each between 0,4 and 0,6 mm.
- the first thickness equals about 0,4 mm and the second thickness equals about 0,5 mm.
- the third thickness of the inner membrane is between 0,8 mm and 3,0 mm, preferably between 0,9 mm and 2,0 mm.
- the fourth thickness of the inner membrane is between 0,30 mm and 0,70 mm, preferably about 0,5 mm.
- the cushion frame has a triangular shape or a saddle shape.
- the cushion frame comprises fixing means for fixing the cushion frame to a respiratory mask. Actually, the cushion frame can be adapted to be removably or permanently connected, via mechanical and/or adhesive fastening, to the mask body. Preferably, the cushion frame includes a base wall structured to be connected to the peripheral wall of mask body, for instance the wall of the cushion frame at its free end can have particular structure, such as a U-, Y-, V-shape or similar, that matches the peripheral border structure of the mask body so as to form together a tight connection. Such structures are well known in the art as taught, for instance, by documents EP-A-1118346, WO-A-9804310, WO-A-2006074513 or EP-A-1841481.
- the inner and outer membranes and the cushion frame are molded in one piece.
- the inner and outer membranes and the cushion frame are made of a resilient, elastomeric material, preferably of silicone.
- the outer membranes comprises a free curved edge projecting toward the interior of the cushion.

The invention also concerns a respiratory mask comprising a mask body with an internal chamber and a gas inlet orifice in fluid communication with said internal chamber, the internal chamber comprising a peripheral border, and the mask body further comprising a cushion according to the present invention that is fixed to the peripheral border of the mask body.

The mask according to the present invention can further comprise one or more of the following additional features:
- the mask is a facial mask.
- the mask further comprises a forehead support, a headgear and/or fixing means for fixing the headgear to the mask body, such as hooks, buckles or similar.
- the forehead support is arranged on a holding arm connected to an expansion part of the mask body, and a rotating knob allows for modification of the angular position of the holding arm when said rotating knob is operated/rotated by a user.
- the expansion part is projecting upwardly and is further integral with the mask body, preferably the expansion part and the mask body are molded in one piece.
- the forehead support comprises one or several pillows of soft material with the pillows coming into contact with the forehead of the user.
- a hollow connector having an internal gas passage, such as an elbow-shape connector, is connected to the gas inlet orifice so as to be in fluid communication with the internal chamber of the mask.
- the hollow connector comprises an anti-asphyxia valve.

In choosing different thicknesses for the nasal bridge region and for the chin region of the outer membrane of the cushion and/or different thicknesses of the inner membrane in the nasal bridge region and in the chin region, it is possible to have a better control of the way the mask cushion fits with the nasal bridge region of the patient. Indeed, in doing so, the outer membrane can better match the nasal bridge region profile thus limiting and minimizing the gas leaks.

An embodiment of a cushion for a facial mask according to the present invention is shown in the enclosed Figures, among which :
- Figure 1 represents a facial mask according to the present invention when positioned on the patient's face,
- Figure 2 shows a cushion according to the present invention,
- Figure 3 represents a cross-sectional view of the cushion of Fig. 2, and
- Figures 4 and 5 are enlarged views of areas A and B of Fig. 3.

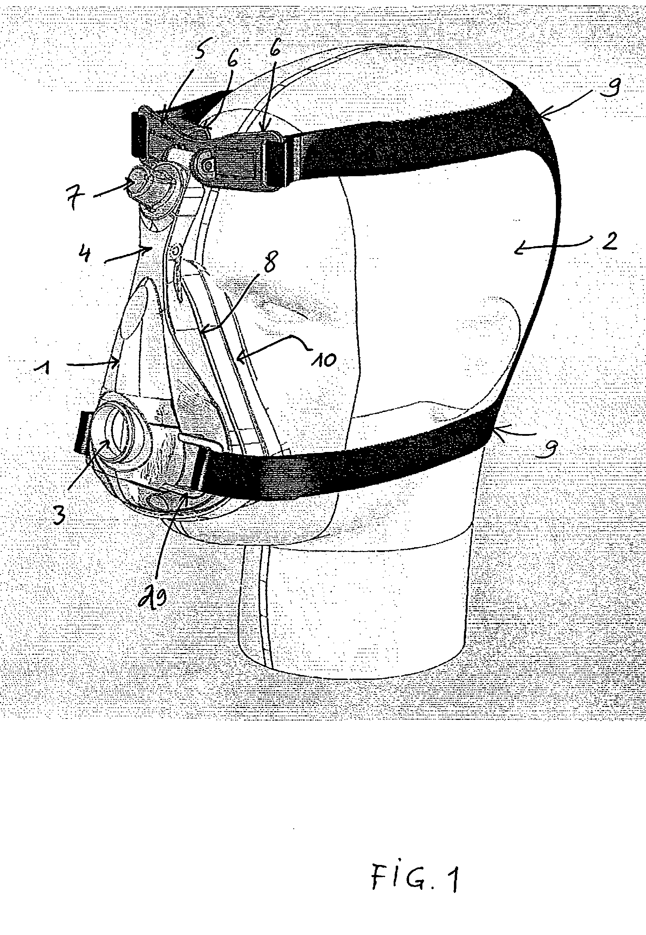

As illustrated in Figure 1, a respiratory facial mask according to the present invention comprises a rigid or semi-rigid hollow shell or mask body 1 defining an internal breathing chamber or volume, wherein respiratory gas, such as air under pressure, is introduced via an inlet port 3 to which is connected a gas feeding line, such a flexible gas conduit, by means of a tubular hollow connector. Preferably, the connector has a general elbow-shape and comprises an internal passage for the gas. The gas inlet orifice 3 is arranged at the center of the mask body 1 and through the mask body wall thereby allowing air under pressure to be introduced in the breathing chamber that receives the nose and mouth of the patient 2.

The mask body 1 is preferably made of a polymer material, such as polycarbonate (PC), polypropylene (PP), ABS, nylon or polystyrene (PS), and is configured so as to be able to receive at least a part of the patient's nose. In other words, the patient introduces his/her nose and mouth into the internal volume of the breathing chamber of the mask body 1 and breathes the pressurized gas contained therein. The mask body 1 has preferably a general triangular or quasi-triangular three-dimensional shape as visible in Figure 1, for example.

The mask body 1 further comprises an expansion part 4 arranged on the top of the mask body 1 and projecting upwardly from said mask body 1, i.e. projecting away from the external surface of the mask body 1. The expansion part 4 as well as the mask body 1 are made of polymeric material as described hereinbefore that is molded in one piece so as to obtain an expansion part 4 that is integral with the rest of the mask body 1.

Further, as represented in Figure 1, a forehead support 5 is connected to the mask body 1 by means of a pivotable holding arm fixed to or carried by either the mask body 1 or the expansion part 4, preferably by the expansion part 4. The forehead support 5 can comprise one or several pillows 6 of soft and comfortable material that come into contact with the patient's forehead.

An acting piece (not visible in Fig. 1) is arranged in a traversing orifice of the expansion part 4 and is mobile, preferably in translation, in said traversing orifice so as to cooperate with the holding arm for pivoting said holding arm, when said acting piece moves in the traversing orifice of the expansion part 4. Further, the forehead support 5 can also pivot with respect to the holding arm 4.

Actually, the backward or forward motion of the acting piece in the traversing orifice is obtained by manual rotation by the user of a rotating knob 7 cooperating with the acting piece, when said rotating knob 7 is manually operated, i.e. turned clockwise or counterclockwise, by the user. The maximum course of the knob 6 is about 360° or less.

Furthermore, in order to ensure a tight positioning of the nasal mask on the patient's face and to increase the comfort for the patient 2, the peripheral border or edge 8 of the mask body 1 comprises a cushion 10 made of soft, resilient, elastomeric material that comes into contact with the patient's face.

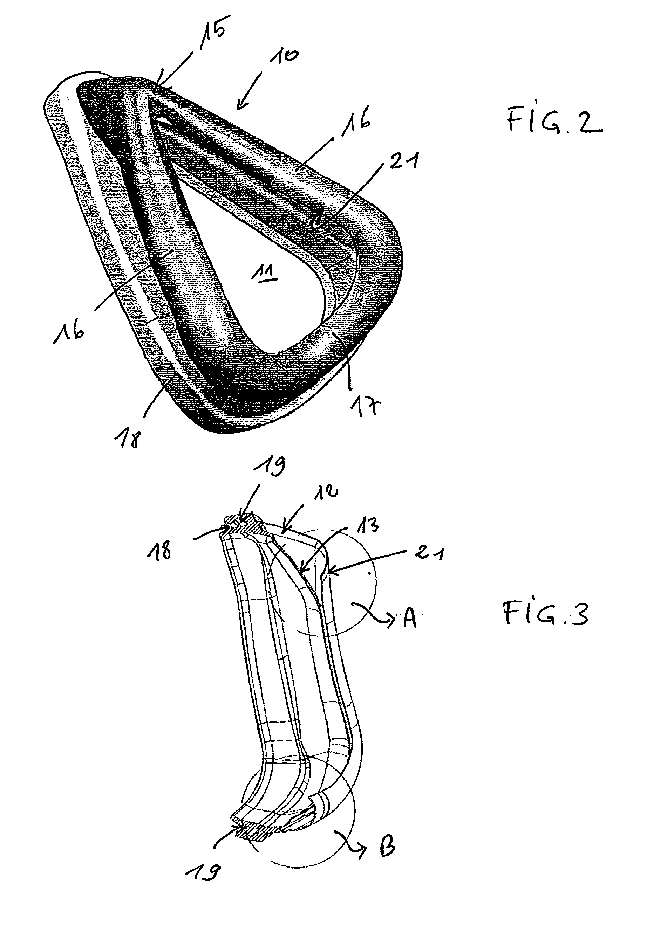

As represented in Figure 2, said cushion 10 has a central aperture 11 for receiving at least a part of the patient's nose. The central aperture 11 of the cushion 10 is located vis a vis with the internal chamber of the mask body 1, i.e. they are facing each other. The cushion 10 arranged on the peripheral border 8 of the internal chamber of the mask body 1 comprises two superimposed flexible membranes 12, 13 ensuring an air tightness so as to minimize air leaks.

Such kinds of cushion 10 and mask body 1 structures are well-known in the art and taught by many documents, such as EP-A-1334742, EP-A-264772, EP-A-956069, EP-A-874667, US-A-2,931,356 or EP-A-1479406.

More precisely, the border 8 and the cushion 10 have a general triangular or saddle-shape structure so as to match the contours of the nasal region, including the upper nasal bridge region, the cheek regions on both sides of the nose, and the lower chin region of the patient 2.

Further, a headgear 9 comprising straps can be connected to the mask body 1, by fixing means 29, such as hooks, buckles or slots, for fixing the straps of the headgear 9. Thereby, the mask is maintained in a desired position on the face of the patient during use of the mask and thus an efficient treatment of sleep disorders, such as OSA or similar disorders, is obtained.

The headgear-fixing means 19 can be made integral with the mask body 1. Such structures are well known in the art and disclosed in many documents such as, for example, EP-A-1334742, EP-A-462701, EP-A-1985327 or EP-A-956069.

The shell or mask body 1 is fluidly linked to a gas supply line, such as a flexible hose or conduit conveying respiratory gas to the mask, by means of a tubular hollow connector, which delivers a respiratory gas, such as air under pressure, into the breathing chamber of the shell 1 through the gas inlet 3 arranged in the mask body 1. Gas under pressure, such as air, can be produced and delivered in a BPAP or CPAP-type apparatus.

Furthermore, the inner and outer membranes 12, 13 are made of flexible material, such as a resilient, elastomeric material like silicone, so that they can fit with the patient's face contours.

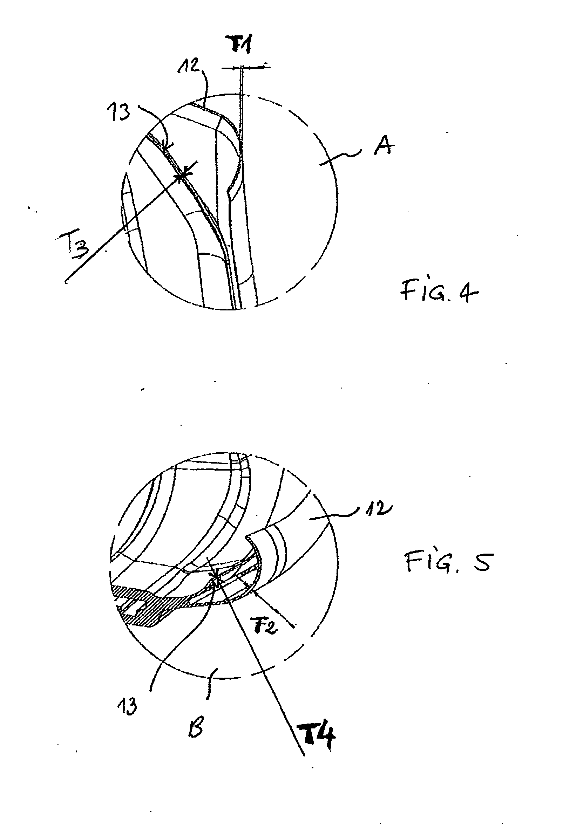

According to the present invention, the outer membrane 12 has, in the nasal bridge region 15, a first thickness T1 that is less than the second thickness T2 of said outer membrane 12 in the chin region 17, as shown in Figures 3 to 5. For instance, the first thickness T1 is of about 0.4 mm, whereas the second thickness T2 is of about 0,50 mm. Using different thicknesses (T1, T2) in the upper nasal bridge region 15 and in the lower chin region 17 as shown in Figures 2 to 5, allows for obtaining a more efficient gas tightness in the upper bridge region 15 as the outer membrane 12 is able to better fit or match the contours of the patient's nasal bridge region 15 when the mask equipped with such a cushion 10 is applied on the patient's face.

Further, the inner membrane 13 has, in the nasal bridge region 15, a first thickness T3 that is more than the fourth thickness T4 of said inner membrane 13 in the chin region 17, as shown in Figures 3 to 5. For instance, the first thickness T3 is of about 1,5 mm, whereas the second thickness T4 is of about 0,5 mm. Using different thicknesses (T3, T4) in the nasal bridge region 15 and in the chin region 17 as shown in Figures 2 to 5, allows for achieving a more comfortable feeling in the chin region 15 as the inner membrane 13 is able to better fit or match the contours of the bottom part of the mouth 17 when the mask equipped with such a cushion 10 is applied on the patient's face.

The cushion frame 10 also comprises fixing means 19, such as a particular configuration (see Fig. 3 for instance) of its peripheral border 18, for fixing the cushion frame 10 to a respiratory mask body 1. Actually, the cushion frame can be adapted to be removably or permanently connected, via mechanical and/or adhesive fastening, to the mask body. Preferably, the cushion frame includes a base wall structured to be connected to the peripheral wall of mask body, for instance the wall of the cushion frame at its free end can comprise a particular structure or configuration, such as a U-, Y-, V-shape or similar, that matches and/or cooperate with the peripheral border structure of the mask body so as to form together a tight connection. Such structures are well known in the art as taught, for instance, by documents EP-A-1118346, WO-A-9804310, WO-A-2006074513 or EP-A-1841481.

The inner and outer membranes 12, 13 and the cushion frame 18 are preferably molded in one-piece and/or made of a resilient, elastomeric material such as silicone.

In addition, the outer membrane 12 advantageously comprises a free curved edge 21 projecting toward the interior of the cushion. Therefore, the width of the outer membrane 12 should be larger than the one of the inner membrane 13 so that said outer membrane 12 can totally cover the inner membrane as shown in Figures 3 to 5.

While the facial mask of the present invention can be used in any manner in which facial masks are necessary, the facial mask of the present invention is particularly useful in a method for treatment of a respiratory disorder or condition, for example, in non-invasive positive pressure ventilation (NPPV) or in a continuous positive airway pressure (CPAP) therapy of sleep disordered breathing (SDB) conditions, such as, for example, obstructive sleep apnea (OSA).

## Claims

1. Cushion (10) for respiratory mask, having a three-dimensional shape with a central passage (11) for receiving at least a part of the nose and the mouth of a user, said cushion (10) comprising at least a nasal bridge region (15), a chin region (17) and two cheek regions (16), which come into contact with the nasal bridge region, the cheek regions and the chin region of the user, respectively, when the cushion (10) is positioned on the user's face, said cushion (10) further comprising an inner membrane (13) and an outer membrane (12), said inner and outer membranes (12, 13) being superimposed so that the outer membrane (12) covers the inner membrane (13) and is in direct contact with the face of the user (2), said inner and outer membranes (12, 13) being made of a flexible material, the outer membrane (12) having, in the nasal bridge region (15) of the cushion, a first thickness (T1) that is less than the second thickness (T2) of said outer membrane (12) in the chin region (17), and the inner membrane (13) having, in the nasal bridge region (15) of the cushion, a third thickness (T3) that is more than the fourth thickness (T4) of said inner membrane (13) in the chin region (17), **characterized in that** the third thickness (T3) is at least 0,8 mm and the fourth thickness (T4) is less than 0,8 mm.

2. Cushion according to Claim 1, **characterized in that** the inner and outer membranes (12, 13) are arranged on a cushion frame (18).

3. Cushion according to any one of the preceding Claims, **characterized in that** the third thickness (T3) of the inner membrane is between 0,8 and 3,0 mm.

4. Cushion according to any one of the preceding Claims, **characterized in that** the third thickness (T3) of the inner membrane is between 0,9 and 2,0 mm.

5. Cushion according to any one of the preceding Claims, **characterized in that** the fourth thickness (T4) of the inner membrane is between 0,3 and 0,7 mm, preferably between 0,4 and 0,5 mm.

6. Cushion according to any one of the preceding Claims, **characterized in that** the first thickness (T1) is between 0,4 and 0,5 mm and/or the second thickness (T2) is between 0,5 and 0,7 mm, preferably between 0,5 and 0,6 mm.

7. Cushion according to any one of the preceding Claims, **characterized in that** the first thickness (T1) equals about 0,4 mm and the second thickness (T2) equals about 0,5 mm.

8. Cushion according to any one of the preceding Claims, **characterized in that** the cushion frame (10) has a triangular shape or a saddle shape.

9. Cushion according to any one of the preceding Claims, **characterized in that** the cushion frame (10) comprises fixing means (19) for fixing the cushion frame (10) to a respiratory mask body (1).

10. Cushion according to any one of the preceding Claims, **characterized in that** the inner and outer membranes (12, 13) and the cushion frame (18) are molded in one piece.

11. Cushion according to any one of the preceding Claims, **characterized in that** the inner and outer membranes (12, 13) and the cushion frame (18) are made of silicone.

12. Cushion according to any one of the preceding Claims, **characterized in that** the outer membranes (12, 13) comprises a free curved edge (21) projecting toward the interior of the cushion (10).

13. Respiratory mask comprising a mask body (1) with an internal chamber and a gas inlet orifice (3) in fluid communication with said internal chamber, the internal chamber comprising a peripheral border (8), and the mask body (1) further comprising a cushion (10) according to any one of the preceding Claims that is fixed to the peripheral border (8) of the mask body (1).

14. Respiratory mask according to claim 13, **characterized in that** the mask is a facial mask.

15. Respiratory mask according to any one of Claims 13 or 14, **characterized in that** it further comprises a forehead support (5), a headgear (9) and/or connecting means (29) for fixing the headgear (9) to the mask body (1).

## Patentansprüche

1. Kissen (10) für eine Atemmaske, das eine dreidimensionale Form mit einem mittigen Durchlass (11) zur Aufnahme wenigstens eines Teils der Nase und des Mundes eines Benutzers aufweist, wobei das Kissen (10) wenigstens einen Nasensattelbereich (15), einen Kinnbereich (17) und zwei Wangenbereiche (16) umfasst, die mit dem Nasensattelbereich, den Wangenbereichen bzw. dem Kinnbereich des Benutzers in Kontakt kommen, wenn das Kissen (10) auf dem Gesicht des Benutzers positioniert wird, wobei das Kissen (10) weiterhin eine innere Membran (13) und eine äußere Membran (12) umfasst, wobei die innere und die äußere Membran (12, 13) einander überlagern, so dass die äußere Membran (12) die innere Membran (13) bedeckt und in direktem Kontakt mit dem Gesicht des Benutzers (2) ist, wobei die innere und die äußere Membran (12, 13) aus einem flexiblen Material gefertigt sind, wobei die äußere Membran (12) in dem Nasensattelbereich (15) des Kissens eine erste Dicke (T1) aufweist, die geringer ist als die zweite Dicke (T2) der äußeren Membran (12) in dem Kinnbereich (17), und die innere Membran (13) in dem Nasensattelbereich (15) des Kissens eine dritte Dicke (T3) aufweist, die größer ist als die vierte Dicke (T4) der inneren Membran (13) in dem Kinnbereich (17), **dadurch gekennzeichnet, dass** die dritte Dicke (T3) wenigstens 0,8 mm beträgt und die vierte Dicke (T4) weniger als 0,8 mm beträgt.

2. Kissen nach Anspruch 1, **dadurch gekennzeichnet, dass** die innere und die äußere Membran (12, 13) auf einem Kissenrahmen (18) angeordnet sind.

3. Kissen nach einem der vorangehenden Ansprüche, **dadurch gekennzeichnet, dass** die dritte Dicke (T3) der inneren Membran zwischen 0,8 und 3,0 mm beträgt.

4. Kissen nach einem der vorangehenden Ansprüche, **dadurch gekennzeichnet, dass** die dritte Dicke (T3) der inneren Membran zwischen 0,9 und 2,0 mm beträgt.

5. Kissen nach einem der vorangehenden Ansprüche, **dadurch gekennzeichnet, dass** die vierte Dicke (T4) der inneren Membran zwischen 0,3 und 0,7 mm, vorzugsweise zwischen 0,4 und 0,5 mm, beträgt.

6. Kissen nach einem der vorangehenden Ansprüche, **dadurch gekennzeichnet, dass** die erste Dicke (T1) zwischen 0,4 und 0,5 mm und/oder die zweite Dicke (T2) zwischen 0,5 und 0,7 mm, vorzugsweise zwischen 0,5 und 0,6 mm, beträgt.

7. Kissen nach einem der vorangehenden Ansprüche, **dadurch gekennzeichnet, dass** die erste Dicke (T1) ca. 0,4 mm beträgt und die zweite Dicke (T2) ca. 0,5 mm beträgt.

8. Kissen nach einem der vorangehenden Ansprüche, **dadurch gekennzeichnet, dass** der Kissenrahmen (10) eine dreieckige Form oder eine Sattelform aufweist.

9. Kissen nach einem der vorangehenden Ansprüche, **dadurch gekennzeichnet, dass** der Kissenrahmen (10) Befestigungsmittel (19) zum Befestigen des Kissenrahmens (10) an einem Atemmaskenkörper (1) umfasst.

10. Kissen nach einem der vorangehenden Ansprüche, **dadurch gekennzeichnet, dass** die innere und die äußere Membran (12, 13) und der Kissenrahmen (18) in einem Stück geformt sind.

11. Kissen nach einem der vorangehenden Ansprüche, **dadurch gekennzeichnet, dass** die innere und die äußere Membran (12, 13) und der Kissenrahmen (18) aus Silikon gefertigt sind.

12. Kissen nach einem der vorangehenden Ansprüche, **dadurch gekennzeichnet, dass** die äußeren Membranen (12, 13) einen freien gebogenen Rand (21) umfassen, der zum Inneren des Kissens (10) hin vorsteht.

13. Atemmaske, umfassend einen Maskenkörper (1) mit einer inneren Kammer und einer Gaseinlassöffnung (3), die in Fließverbindung mit der inneren Kammer steht, wobei die innere Kammer eine periphere Begrenzung (8) umfasst und der Maskenkörper (1) weiterhin ein Kissen (10) nach einem der vorangehenden Ansprüche umfasst, das an der peripheren Begrenzung (8) des Maskenkörpers (1) befestigt ist.

14. Atemmaske nach Anspruch 13, **dadurch gekennzeichnet, dass** die Maske eine Gesichtsmaske ist.

15. Atemmaske nach einem der Ansprüche 13 oder 14, **dadurch gekennzeichnet, dass** sie weiterhin eine Stirnstütze (5), eine Kopfhalterung (9) und/oder Verbindungsmittel (29) zum Befestigen der Kopfhalterung (9) am Maskenkörper (1) umfasst.

## Revendications

1. Coussinet (10) pour masque respiratoire, de forme tridimensionnelle avec un passage central (11) pour recevoir au moins une partie du nez et de la bouche d'un utilisateur, ledit coussinet (10) comprenant au moins une région de voûte nasale (15), une région de menton (17) et deux régions de joue (16), qui viennent en contact avec la région de voûte nasale, les régions de joue et la région de menton de l'utilisateur, respectivement, lorsque le coussinet (10) est positionné sur le visage de l'utilisateur, ledit coussinet (10) comprenant en outre une membrane intérieure (13) et une membrane extérieure (12), lesdites membranes intérieure et extérieure (12, 13) étant superposées de sorte que la membrane extérieure (12) couvre la membrane intérieure (13) et soit en contact direct avec le visage de l'utilisateur (2), lesdites membranes intérieure et extérieure (12, 13) étant en un matériau flexible, la membrane extérieure (12) ayant, dans la région de voûte nasale (15) du coussinet, une première épaisseur (T1) qui est inférieure à la deuxième épaisseur (T2) de ladite membrane extérieure (12) dans la région de menton (17), et la membrane intérieure (13) ayant, dans la région de voûte nasale (15) du coussinet, une troisième épaisseur (T3) qui est supérieure à la quatrième épaisseur (T4) de ladite membrane intérieure (13) dans la région de menton (17), **caractérisé en ce que** la troisième épaisseur (T3) est d'au moins 0,8 mm et que la quatrième épaisseur (T4) est inférieure à 0,8 mm.

2. Coussinet selon la revendication 1, **caractérisé en ce que** les membranes intérieure et extérieure (12, 13) sont agencées sur un cadre de coussinet (18).

3. Coussinet selon l'une quelconque des revendications précédentes, **caractérisé en ce que** la troisième épaisseur (T3) de la membrane intérieure est comprise entre 0,8 et 3,0 mm.

4. Coussinet selon l'une quelconque des revendications précédentes, **caractérisé en ce que** la troisième épaisseur (T3) de la membrane intérieure est comprise entre 0,9 et 2,0 mm.

5. Coussinet selon l'une quelconque des revendications précédentes, **caractérisé en ce que** la quatrième épaisseur (T4) de la membrane intérieure est comprise entre 0,3 et 0,7 mm, de préférence entre 0,4 et 0,5 mm.

6. Coussinet selon l'une quelconque des revendications précédentes, **caractérisé en ce que** la première épaisseur (T1) est comprise entre 0,4 et 0,5 mm et/ou la deuxième épaisseur (T2) est comprise entre 0,5 et 0,7 mm, de préférence entre 0,5 et 0,6 mm.

7. Coussinet selon l'une quelconque des revendications précédentes, **caractérisé en ce que** la première épaisseur (T1) est égale à environ 0,4 mm et la deuxième épaisseur (T2) est égale à environ 0,5 mm.

8. Coussinet selon l'une quelconque des revendications précédentes, **caractérisé en ce que** le cadre de coussinet (10) a une forme triangulaire ou une forme de selle.

9. Coussinet selon l'une quelconque des revendications précédentes, **caractérisé en ce que** le cadre de coussinet (10) comprend un moyen de fixation (19) pour fixer le cadre de coussinet (10) à un corps de masque respiratoire (1).

10. Coussinet selon l'une quelconque des revendications précédentes, **caractérisé en ce que** les membranes intérieure et extérieure (12, 13) et le cadre de coussinet (18) sont moulés en une seule pièce.

11. Coussinet selon l'une quelconque des revendications précédentes, **caractérisé en ce que** les membranes intérieure et extérieure (12, 13) et le cadre de coussinet (18) sont en silicone.

12. Coussinet selon l'une quelconque des revendications précédentes, **caractérisé en ce que** les membranes extérieures (12, 13) comprennent un bord incurvé libre (21) dépassant vers l'intérieur du coussinet (10).

13. Masque respiratoire comprenant un corps de masque (1) doté d'une chambre interne et d'un orifice d'admission de gaz (3) en communication fluidique avec ladite chambre interne, la chambre interne comprenant une bordure périphérique (8), et le corps de masque (1) comprenant en outre un coussinet (10) selon l'une quelconque des revendications précédentes, qui est fixé à la bordure périphérique (8) du corps de masque (1).

14. Masque respiratoire selon la revendication 13, **caractérisé en ce que** le masque est un masque pour le visage.

15. Masque respiratoire selon l'une quelconque des revendications 13 ou 14, **caractérisé en ce qu'**il comprend en outre un support de front (5), un harnais (9) et/ou un moyen de raccordement (29) pour fixer le harnais (9) au corps de masque (1).
